# EUROPEAN PATENT SPECIFICATION

(11) **EP 2 561 837 B1**
(45) Date of publication and mention of the grant of the patent: **06.06.2018**
(21) Application number: 11771568.0
(22) Date of filing: 19.04.2011
(51) Int. Cl.: A61F 2/90, A61L 27/04, A61L 31/00, A61F 2/852, A61F 2/915, A61F 2/06, A61F 2/856

(54) **STENT FOR BIFURCATED VESSEL**
STENT FÜR EIN GEGABELTES GEFÄSS
ENDOPROTHÈSE POUR VAISSEAU BIFURQUÉ

(30) Priority: 20.04.2010 CN 201010151612
(43) Date of publication of application: 27.02.2013
(73) Proprietor: Shanghai MicroPort Medical (Group) Co., Ltd., Pudong New Area Shanghai Shanghai 201203 (CN)
(72) Inventor: ZHANG, Dadong, Shanghai 201203 (CN); LI, Yan, Shanghai 201203 (CN); WANG, Changchun, Shanghai 201203 (CN); TANG, Zhirong, Shanghai 201203 (CN); LUO, Qiyi, Shanghai 201203 (CN)
(74) Representative: Dantz, Jan Henning
(86) International application number: PCT/CN2011/072972
(87) International publication number: WO 2011/131115

(56) References cited:
- WO-A1-00/57813
- WO-A1-96/28116
- WO-A1-2007/073413
- WO-A2-2008/091515
- CN-A- 1 194 577
- CN-Y- 2 643 861
- CN-Y- 200 948 183
- CN-Y- 200 966 659
- US-A- 5 893 887
- US-A- 5 911 754
- US-A1- 2004 068 314
- US-A1- 2007 270 769

## Description

### Technical Field

The present application relates to the implantable medical device field and in particular to a stent for a bifurcated vessel.

### Background Art

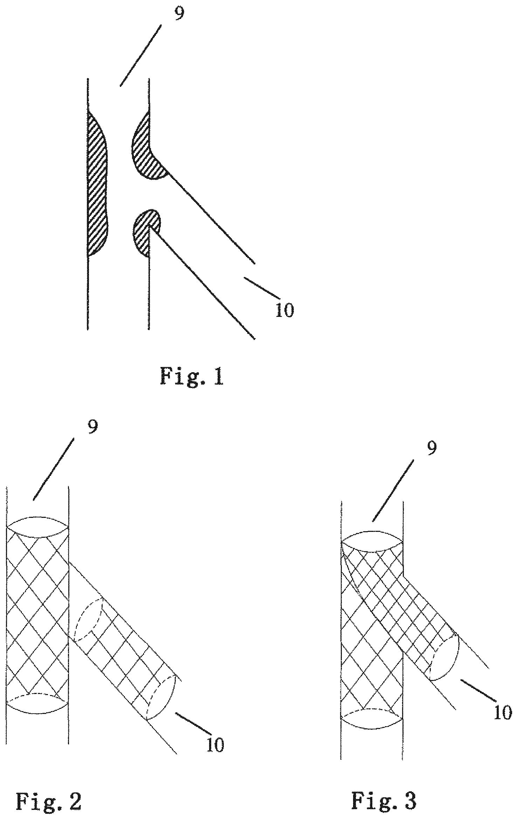

A stent for a vessel is generally a drug eluting stent for treating vascular stenosis. The stent for a vessel implanted in the human body assists the lesion vessel in recovering by supporting the lesion vessel. Meanwhile, the stent for a vessel can also release a drug on the stent to the vascular wall in contact therewith to inhibit growth of cells of the vascular wall and reduce the incidence rate of vascular restenosis.
In the clinical practice, with respect to many patients, the vascular stenosis does not occur in only one place but in multiple places in the vessel. The bifurcated lesion vessel is a common multiple artery stenosis. As shown by the shaded portions in Fig. 1, the vascular lesion sites are positioned at the intersection of a main vessel 1 and a branch vessel 2.

In the process of developing the present application, the inventors find that at least the following problems exist in the prior art: 1) as shown in Fig. 2, an existing stent for a branch vessel has a structure with both ends flush, and cannot completely and sufficiently cover the vessel at the lesion sites when treating the bifurcated lesion vessel, thus influencing the treating effect; 2) as shown in Fig. 3, an existing branch vessel adopts the "crush" technique, but the stent for a branch vessel and the stent for a main vessel overlap too much, which results in the amount of the implanted metal being too much, whereby thrombus is likely to be formed at the intersection of the vessels.

Document WO 2007/073413 A1 describes a stent with serpentine bands and an overall envelope shape of increasing and/or decreasing longitudinal extent in a proximal direction. The stent is designed to be used in superficial femoral artery.

Document US 2007/0270769 A1 designed for bifurcated vessels. The stent is partially cut in a longitudinal direction and the cut-off lobes are bent outwards to point into different directions.

Document WO 96/28116 A1 discloses a stent with at least one oblique end which is disposed at an oblique angle relative to the lumen of the vessel it is applied to. The oblique ends are achieved by a varying number of annular units of the stent along its circumference. Such a stent is particularly suitable for bifurcations. Stents having normal ends tend to block branch vessels, which is avoided by the oblique end.

Document WO 00/057813 A1 also describes a stent with at least one oblique end. The oblique end is achieved by a varying geometric structure and size of the annular units of the stent along its circumference. The stent is preferable implanted by using a balloon which carries a radio-opaque marker.

### Summary of the Invention

In order to solve the above technical problem, the embodiments of the present application provide a stent for a bifurcated vessel to solve the problem that the existing stents for a bifurcated vessel cannot completely cover the lesion site or overlap at the lesion site after being implanted in the human body. The technical solutions are as follows:
A stent for a bifurcated vessel, comprising a stent body with two open ends, the stent body comprising: multiple sets of annular units having multiple undulating rods; and connecting rods positioned between adjacent annular units and used to connect the adjacent annular units, wherein the structure of at least one open end of the stent body is a slope structure.

The number of the undulating rods in the multiple sets of annular units forming the slope structure decreases in turn in a direction from a middle part of the stent body to the open end having the slope structure.

Alternatively or additionally, the compactness of the undulating rods in the multiple sets of annular units forming the slope structure increases in turn in a direction from a middle part of the stent body to the open end having the slope structure.

Furthermore, at least four developable marks are further provided around the slope surface of the slope structure, wherein at least two developable marks are respectively provided at the top and bottom of the slope surface of the slope structure, and at least two other developable marks are symmetrically provided on both sides of the slope surface along an axial center line.

Preferably, the axial length of the slope structure is 1 - 7mm.

Preferably, the axial length of the slope structure is 4 ~ 6mm.

Preferably, an included angle between the slope surface of the slope structure and the axial direction of the stent body is between 0 degree and 90 degrees.

Preferably, the included angle between the slope surface of the slope structure and the axial direction of the stent body is 45 degrees.

Preferably, the stent for a bifurcated vessel is a stent for a bifurcated coronary artery.

Preferably, the diameter of the stent for a bifurcated coronary artery is 2.25mm ~ 4.0mm.

Preferably, the material of the stent body is a stainless steel, a cobalt-chromium alloy, a nickel-based alloy, a degradable magnesium alloy or a polymer material having good biological compatibility and mechanical characteristics.

Preferably, the top of the slope structure can be also of a smooth arc shape or a flush shape.

In the technical solutions provided by the embodiments of the present application, at at least one end of the stent for a bifurcated vessel, the number of the undulating rods in each set of annular unit is decreased in turn, or the compactness of the undulating rods in each set of annular unit is increased in turn, or the two are performed simultaneously, in a direction from the middle part of the stent body to the open end having the slope structure, to shorten the length of each set of annular unit in turn to form a slope structure. The angle of the slope structure matches with the bifurcation angle of the branch vessel. Thus, the stent for a bifurcated vessel can completely and sufficiently cover the vessel at the lesion site and will not overlap the stent for a main vessel after being implanted in the lesion site of the bifurcated vessel of the human body.

In addition, at least four developable marks are provided around the slope surface of the slope structure of the stent for a bifurcated vessel. In the at least four developable marks, at least two developable marks are respectively provided at the top and bottom of the slope structure, and at least two other developable marks are symmetrically provided on both sides of the slope surface of the slope structure along an axial center line. In the delivering process, the doctor can clearly distinguish the slope surface of the slope structure of the stent for a bifurcated vessel according to the development positions of the developable marks, and then the doctor rotates the stent by rotating a balloon dilatation catheter, so that the slope surface of the slope structure can join the main vessel, and then releases and dilates the stent. Thus, this stent for a bifurcated vessel can be also located accurately in the delivering and releasing process to facilitate the surgical procedure by the doctor.

### Brief Description of the Drawings

In order to describe the technical solutions in the embodiments of the present application or the prior art more clearly, the figures to be used in the descriptions of the embodiments or the prior art will be briefly introduced below. It is obvious that the figures in the descriptions below only relate to some embodiments recorded in the present application, and those skilled in the art can also obtain other figures according to these figures without making inventive efforts.
Fig. 1 is a schematic diagram of the lesion sites of a common bifurcated lesion vessel;
Fig. 2 is a schematic diagram of operation of the exiting stent for a branch vessel with both ends flush;
Fig. 3 is a schematic diagram of operation of the existing stent for a branch vessel adopting the "crush" technique;
Fig. 4 is a schematic diagram of an anatomical structure of a stent for a bifurcated vessel provided by an embodiment of the present application;
Fig. 5 is a schematic diagram of another preferable anatomical structure of a stent for a bifurcated vessel provided by an embodiment of the present application;
Fig. 6(a) and Fig. 6(b) are schematic diagrams of the developable marks of the stent for a bifurcated vessel provided by an embodiment of the present application;
Fig. 7 is a schematic diagram of the structure where the stent for a bifurcated vessel is fixed to the balloon dilatation catheter provided by an embodiment of the present application;
Fig. 8 is a schematic diagram of one operation of the stent for a bifurcated vessel provided by an embodiment of the present application; and
Fig. 9 is a schematic diagram of another operation of the stent for a bifurcated vessel provided by an embodiment of the present application.

### Detailed Description

In order to make those skilled in the art better understand the technical solutions in the present application, the technical solutions in the embodiments of the present application will be described clearly and completely below by taking the figures in the embodiments of the present application into consideration. Obviously, the described embodiments are only parts of the embodiments of the present application rather than all the embodiments. All the other embodiments obtained by those skilled in the art based on the embodiments in the present application without making inventive efforts should belong to the scope of protection of the present application.

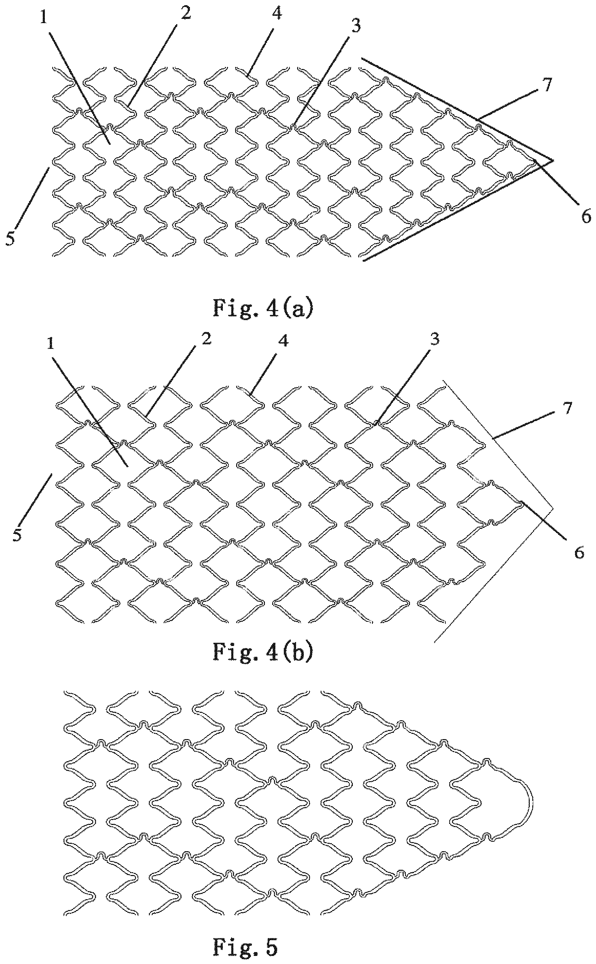

Fig. 4 is a schematic diagram of an anatomical structure of a stent for a bifurcated vessel provided by an embodiment of the present application.

As shown in Fig. 4, a stent body 1 of the stent for a bifurcated vessel comprises multiple sets of annular units 2 and connecting rods 3, each set of annular unit 2 being formed by connection of multiple undulating rods 4, and the connecting rods 3 being positioned between adjacent annular units 2 and used to connect the adjacent annular units 2. The stent body 1 has two open ends. The structure of the first open end 5 is a flush and symmetrical structure. Near the second open end 6, the length of each set of annular unit 2 is shortened by decreasing the number of the undulating rods 4 in each set of annular unit 2, or by increasing the compactness of the undulating rods 4 in each set of annular unit 2, or by performing the two simultaneously, in a direction from the middle part of the stent body 1 to the open end 6. A slope opening 7 is formed at the second open end 6, and in this case, the structure at the second open end 6 forms a slope structure taking the slope opening 7 as the slope surface by curling up the anatomical structure shown in Fig. 4 or Fig. 5. In the other embodiments, the stent body 1 of the stent for a bifurcated vessel can be of the slope structure at both of the open ends.

The bifurcation angle of the vessel inside the human body is generally between 0 degree to 90 degrees, and the bifurcation angles of most of the vessels branched from the bifurcation site of the vessel are about 45 degrees. In the embodiments of the present application, the axial length of the slope structure is set to 1 ~ 7mm, and preferably 4 ~ 6mm. Based on the set axial length of the slope structure, the angle of the slope structure, i.e. the angle between the slope surface surrounded by the slope opening 7 and the axial direction of the stent body 1, can be changed. The angel between the slope opening 7 and the axial direction of the stent body 1 can be changed by decreasing in turn the number of the undulating rods 4 in each set of annular unit 2 at one end of the stent body 1 and adjusting the length of each set of annular unit 2. At the production of the stent for a bifurcated vessel, the angle of the slope structure can be flexibly designed between 0 degree and 90 degrees so as to match with the vessels of different bifurcation angles to meet different requirements for the operation according to the condition of the bifurcation of the vessel at the application site. As shown in Fig. 4(a) and Fig. 4(b), Fig. 4(a) is a schematic diagram of the anatomical structure of the stent for a bifurcated vessel having angle of 45 degrees, and Fig. 4(b) is a schematic diagram of the anatomical structure of the stent for a bifurcated vessel having angle of 60 degrees. In an embodiment of the present application, the angle of the slope structure is preferably designed as 45 degrees, for this angle can cover most of the bifurcation angels of the vessels branched from the bifurcation site of the main vessel.

In the actual surgical application, in order to prevent the top (the tip close to the open end 6 is the top) of the slope opening 7 of the stent for a bifurcated vessel from piercing or scratching the inner wall of the vessel when being implanted, the top of the slope opening 7 is generally processed, and the top of the slope opening 7 is designed as a smooth arc shape or a flush shape, as shown in Fig. 5.

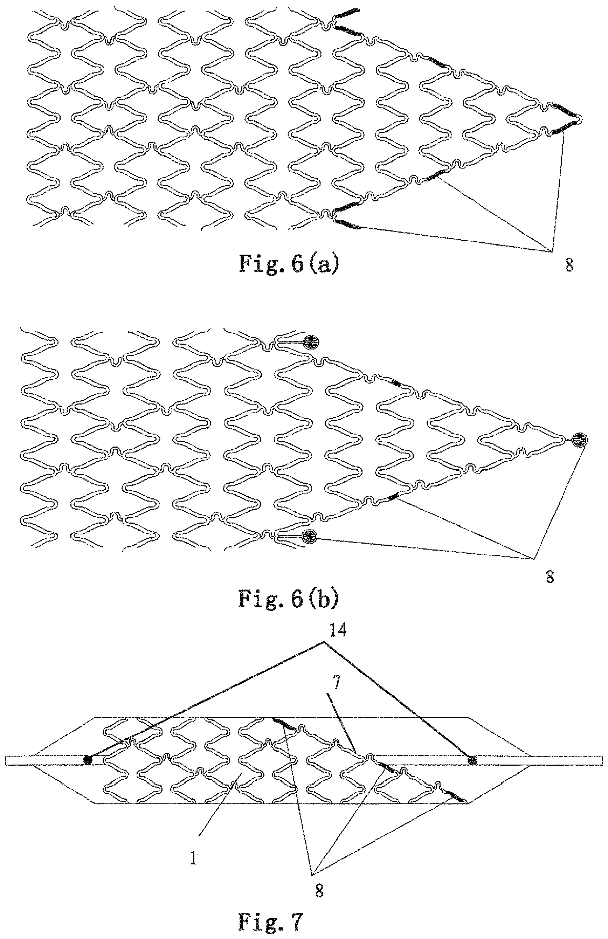

Fig. 6(a) and Fig. 6(b) are schematic diagrams of a developable mark of the stent for a bifurcated vessel provided by an embodiment of the present application.

As shown in Fig. 6(a) and Fig. 6(b), the stent for a bifurcated vessel further comprises developable marks 8, which can be developable films coated or plated on the undulating rods 4 of the stent body 1. As shown in the figures, at least four developable marks 8 are provided, wherein at least two developable marks are respectively provided on the top and bottom (the position farthest from the top in the slope surface surrounded by the slope opening 7 is the bottom) of the slope surface surrounded by the slope opening 7, and at least two other developable marks are symmetrically provided on both sides of the slope surface surrounded by the slope opening 7 along an axial center line for displaying the position of the stent for a bifurcated vessel in the surgical delivery, so that the doctor can distinguish the slope surface surrounded by the slope opening 7 in a more accurate manner and can accurately locate the stent for a bifurcated vessel during the operation. In an embodiment of the present application, the developable marks 8 can be also fixed to the stent body 1 in an embedding or winding manner. As shown in Fig. 6(a), the developable marks 8 can be developable wires wound on the undulating rods 4 of the stent body 1, and as shown in Fig. 6(b), the developable marks 8 can be developable sheet metals embedded into the undulating rods 4 of the stent body 1.

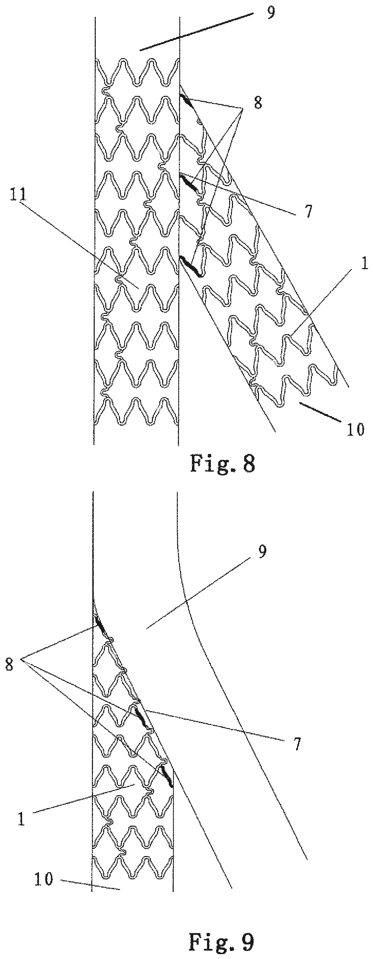

Fig. 8 is a schematic diagram of one operation of the stent for a bifurcated vessel provided by an embodiment of the present application.

As shown in Fig. 8, the lesion of vascular stenosis occurs to both the main vessel 9 and the branch vessel 10, and the reference sign 11 denotes the stent for a main vessel. The stent for a bifurcated vessel is to be used along with a balloon dilatation catheter 12 when being implanted. As shown in Fig. 7, the stent for a bifurcated vessel is firstly crimped on a balloon 13 of the balloon dilatation catheter 12 before being implanted; then the balloon dilatation catheter 12 is made to enter the branch vessel 10 from the main vessel 9 according to developable points 14 on the balloon 13; then the balloon dilatation catheter 12 is rotated according to the development positions of the developable marks 8 provided around the slope surface surrounded by the slope structure 7 in the stent body 1, so that the slope surface surrounded by the slope opening 7 in the stent body 1 joints the main vessel 9, i.e., the slope opening 7 completely contacts the main vessel 9; and finally the stent for a bifurcated vessel is released and dilated to completely cover the lesion site of the vessel, as shown in Fig. 8, to thereby achieve the implanting process.

Fig. 9 is a schematic diagram of another operation of the stent for a bifurcated vessel provided by an embodiment of the present application.

As shown in the figure, the lesion of vascular stenosis occurs to the branch vessel 10 only, and the main vessel 9 is normal. The implanting process of the stent for a bifurcated vessel is the same as the above implanting process, and the state after the implantation is as shown in Fig. 9.

In the embodiments of the present application, at at least one end of the stent for a bifurcated vessel, the number of the undulating rods in each set of annular unit is decreased in turn, or the compactness of the undulating rods in each set of annular unit is increased in turn, or the two are performed simultaneously, in a direction from the middle part of the stent body to the open end having the slope structure, to shorten the length of each set of annular unit in turn to form a slope structure. The angle of the slope structure matches with the bifurcation angle of the branch vessel. Thus, the stent for a bifurcated vessel can completely and sufficiently cover the vessel at the lesion site and will not overlap the stent for a main vessel after being implanted in the lesion site of the bifurcated vessel of the human body.

In addition, at least four developable marks are provided around the slope surface of the slope structure of the stent for a bifurcated vessel. In the at least four developable marks, at least two developable marks are respectively provided at the top and bottom of the slope structure, and at least two other developable marks are symmetrically provided on both sides of the slope surface of the slope structure along an axial center line. In the delivering process, the doctor can clearly distinguish the slope surface of the slope structure of the stent for a bifurcated vessel according to the development positions of the developable marks, and then the doctor rotates the stent by rotating a balloon dilatation catheter, so that the slope surface of the slope structure can joint the main vessel, and then releases and dilates the stent. Thus, this stent for a bifurcated vessel can be also located accurately in the delivering and releasing process to facilitate the surgical procedure by the doctor.

The above contents are only preferred embodiments of the present invention and enable those skilled in the art to understand or achieve the present invention. Multiple amendments to these embodiments are obvious to those skilled in the art, and general principles defined in this application can be achieved in the other embodiments in case of not breaking away from the scope of the present invention. Thus, the present invention will be not limited to these embodiments shown in this application, but shall accord with the widest scope consistent with the principles and novel characteristics disclosed by this application.

## Claims

1. A stent for a bifurcated vessel, comprising a stent body (1) with two open ends, wherein the stent body (1) comprises: multiple sets of annular units (2) having multiple undulating rods (4) ; and connecting rods (3) positioned between adjacent annular units (2) and used to connect the adjacent annular units (2), wherein the structure of at least one open end of the stent body (1) is a slope structure, and wherein the number of the undulating rods (4) in the multiple sets of annular units (2) forming the slope structure decreases in turn and/or the compactness of the undulating rods (4) increases in turn, in a direction from a middle part of the stent body (1) to the open end having the slope structure, **characterized in that** at least four developable marks are further provided around a slope surface of the slope structure, wherein at least two developable marks are respectively provided at the top and bottom of the slope surface of the slope structure, and at least two other developable marks are symmetrically provided on both sides of the slope surface along an axial center line.

2. The stent for a bifurcated vessel according to claim 1, **characterized in that** the axial length of the slope structure is 1~7mm.

3. The stent for a bifurcated vessel according to claim 2, **characterized in that** the axial length of the slope structure is 4~6mm.

4. The stent for a bifurcated vessel according to claim 3, **characterized in that** an included angle between a slope surface of the slope structure and the axial direction of the stent body (1) is between 0 degree and 90 degrees.

5. The stent for a bifurcated vessel according to claim 4, **characterized in that** the included angle between the slope surface of the slope structure and the axial direction of the stent body (1) is 45 degrees.

6. The stent for a bifurcated vessel according to claim 1, **characterized in that** the stent for a bifurcated vessel is a stent for a bifurcated coronary artery.

7. The stent for a bifurcated vessel according to claim 6, **characterized in that** the diameter of the stent for a bifurcated coronary artery is 2.25mm~4.0mm.

8. The stent for a bifurcated vessel according to claim 7, **characterized in that** the material of the stent body (1), having good biological compatibility and mechanical characteristics, is a stainless steel, a cobalt chromium alloy, a nickel based alloy, a degradable magnesium alloy or a polymer material.

9. The stent for a bifurcated vessel according to claim 1, **characterized in that** a top of the slope structure can be also of a smooth arc shape or a flush shape.

## Patentansprüche

1. Stent für ein gegabeltes Gefäß, umfassend einen Stentkörper (1) mit zwei offenen Enden, wobei der Stentkörper (1) umfasst: mehrere Sätze von ringförmigen Einheiten (2), die mehrere wellenförmige Stäbe (4) aufweisen; und Verbindungsstäbe (3), die zwischen benachbarten ringförmigen Einheiten (2) positioniert sind und dazu verwendet werden, die benachbarten ringförmigen Einheiten (2) miteinander zu verbinden, wobei die Struktur von wenigstens einem offenen Ende des Stentkörpers (1) eine Neigungsstruktur ist, und wobei in einer Richtung von einem mittleren Teil des Stentkörpers (1) zum offenen Ende mit der Neigungsstruktur die Anzahl der wellenförmigen Stäbe (4) in den mehreren Sätzen von ringförmigen Einheiten (2), welche die Neigungsstruktur bilden, nacheinander abnimmt und/oder die Kompaktheit der wellenförmigen Stäbe (4) nacheinander zunimmt, **dadurch gekennzeichnet, dass** ferner wenigstens vier entwickelbare Markierungen um eine Neigungsfläche der Neigungsstruktur vorgesehen sind, wobei wenigstens zwei entwickelbare Markierungen an der Oberseite bzw. an der Unterseite der Neigungsfläche der Neigungsstruktur vorgesehen sind und wenigstens zwei andere entwickelbare Markierungen symmetrisch an beiden Seiten der Neigungsfläche entlang einer axialen Mittellinie vorgesehen sind.

2. Stent für ein gegabeltes Gefäß nach Anspruch 1, **dadurch gekennzeichnet, dass** die axiale Länge der Neigungsstruktur 1 bis 7 mm beträgt.

3. Stent für ein gegabeltes Gefäß nach Anspruch 2, **dadurch gekennzeichnet, dass** die axiale Länge der Neigungsstruktur 4 bis 6 mm beträgt.

4. Stent für ein gegabeltes Gefäß nach Anspruch 3, **dadurch gekennzeichnet, dass** ein eingeschlossener Winkel zwischen einer Neigungsfläche der Neigungsstruktur und der axialen Richtung des Stentkörpers (1) zwischen 0 Grad und 90 Grad beträgt.

5. Stent für ein gegabeltes Gefäß nach Anspruch 4, **dadurch gekennzeichnet, dass** der eingeschlossene Winkel zwischen der Neigungsfläche der Neigungsstruktur und der axialen Richtung des Stentkörpers (1) 45 Grad beträgt.

6. Stent für ein gegabeltes Gefäß nach Anspruch 1, **dadurch gekennzeichnet, dass** der Stent für ein gegabeltes Gefäß ein Stent für eine gegabelte Koronararterie ist.

7. Stent für ein gegabeltes Gefäß nach Anspruch 6, **dadurch gekennzeichnet, dass** der Durchmesser des Stents für eine gegabelte Koronararterie 2,25 mm bis 4,0 mm beträgt.

8. Stent für ein gegabeltes Gefäß nach Anspruch 7, **dadurch gekennzeichnet, dass** das Material des Stentkörpers (1), das eine gute biologische Verträglichkeit und mechanische Eigenschaften aufweist, ein rostfreier Stahl, eine Kobalt-Chrom-Legierung, eine Legierung auf Nickelbasis, eine abbaubare Magnesiumlegierung oder ein Polymermaterial ist.

9. Stent für ein gegabeltes Gefäß nach Anspruch 1, **dadurch gekennzeichnet, dass** eine Oberseite der Neigungsstruktur auch eine glatte Bogenform oder eine bündige Form aufweisen kann.

## Revendications

1. Extenseur vasculaire pour un vaisseau bifurqué, comprenant un corps d'extenseur (1) ouvert aux deux extrémités, lequel corps d'extenseur (1) comprend plusieurs ensembles d'unités annulaires (2) possédant plusieurs tiges ondulées (4), ainsi que des tiges de liaison (3) placées entre des unités annulaires (2) adjacentes et servant à relier les unités annulaires (2) adjacentes, la structure d'au moins une extrémité ouverte du corps d'extenseur (1) étant une structure oblique, et le nombre de tiges ondulées (4) dans les plusieurs ensembles d'unités annulaires (2) qui forment la structure oblique diminuant successivement et/ou la densité des tiges ondulées (4) augmentant successivement dans une direction allant du milieu du corps d'extenseur (1) à l'extrémité ouverte munie de la structure oblique, **caractérisé en ce qu'**au moins quatre marques développables sont prévues autour d'une surface oblique de la structure oblique, au moins deux marques développables étant prévues respectivement en haut et en bas de la surface oblique de la structure oblique, et au moins deux autres marques développables étant prévues de façon symétrique des deux côtés de la surface oblique le long d'une ligne axiale centrale.

2. Extenseur vasculaire pour un vaisseau bifurqué selon la revendication 1, **caractérisé en ce que** la longueur axiale de la structure oblique est de 1 à 7 mm.

3. Extenseur vasculaire pour un vaisseau bifurqué selon la revendication 2, **caractérisé en ce que** la longueur axiale de la structure oblique est de 4 à 6 mm.

4. Extenseur vasculaire pour un vaisseau bifurqué selon la revendication 3, **caractérisé en ce qu'**un angle formé entre une surface oblique de la structure oblique et la direction axiale du corps d'extenseur (1) est compris entre 0° et 90°.

5. Extenseur vasculaire pour un vaisseau bifurqué selon la revendication 4, **caractérisé en ce que** l'angle formé entre la surface oblique de la structure oblique et la direction axiale du corps d'extenseur (1) est de 45°.

6. Extenseur vasculaire pour un vaisseau bifurqué selon la revendication 1, **caractérisé en ce que** l'extenseur vasculaire pour un vaisseau bifurqué est un extenseur vasculaire pour une artère coronaire bifurquée.

7. Extenseur vasculaire pour un vaisseau bifurqué selon la revendication 6, **caractérisé en ce que** le diamètre de l'extenseur vasculaire pour une artère coronaire bifurquée est de 2,25 mm à 4,0 mm.

8. Extenseur vasculaire pour un vaisseau bifurqué selon la revendication 7, **caractérisé en ce que** le matériau du corps d'extenseur (1), qui a une bonne biocompatibilité et de bonnes propriétés mécaniques, est un acier inoxydable, un alliage de cobalt et de chrome, un alliage de nickel, un alliage de magnésium dégradable ou un matériau polymère.

9. Extenseur vasculaire pour un vaisseau bifurqué selon la revendication 1, **caractérisé en ce que** le haut de la structure oblique peut aussi avoir une forme lisse en arc de cercle ou une forme en affleurement.
